# EUROPEAN PATENT SPECIFICATION

(11) **EP 4 047 011 B1**
(45) Date of publication and mention of the grant of the patent: **02.07.2025**
(21) Application number: 20877037.0
(22) Date of filing: 22.09.2020
(51) Int. Cl.: C07K 14/00, A61K 38/00, A61P 31/04

(54) **HYBRID ANTIMICROBIAL PROTEIN HAVING STRONG BACTERICIDAL EFFECT, AND APPLICATION THEREOF**
HYBRIDES ANTIMIKROBIELLES PROTEIN MIT STARKER BAKTERIZIDER WIRKUNG UND DESSEN VERWENDUNG
PROTÉINE ANTIMICROBIENNE HYBRIDE À FORT EFFET BACTÉRICIDE, ET SON APPLICATION

(30) Priority: 14.10.2019 CN 201910975220
(43) Date of publication of application: 24.08.2022
(73) Proprietor: Jiangxi Yuansheng Biological Technology Co., Ltd., Yichun Jiangxi 330600 (CN)
(72) Inventor: LU, Hairong, Shanghai 201206 (CN); HUANG, Qingshan, Shanghai 201206 (CN); LI, Guodong, Shanghai 201206 (CN); HUANG, Jinjiang, Shanghai 201206 (CN); ZHAO, Xiaowei, Shanghai 201206 (CN); LU, Wanying, Shanghai 201206 (CN)
(74) Representative: Canzler & Bergmeier Patentanwälte Partnerschaft mbB
(86) International application number: PCT/CN2020/116756
(87) International publication number: WO 2021/073372

(56) References cited:
- WO-A1-2018/185634
- CN-A- 110 227 145
- US-A1- 2003 018 990
- OLIVEIRA HUGO ET AL: "Structural and Enzymatic Characterization of ABgp46, a Novel Phage Endolysin with Broad Anti-Gram-Negative Bacterial Activity", FRONTIERS IN MICROBIOLOGY, vol. 7, 26 February 2016 (2016-02-26), XP093083621, DOI: 10.3389/fmicb.2016.00208
- GERSTMANS HANS ET AL: "Synthetic biology of modular endolysins", BIOTECHNOLOGY ADVANCES., vol. 36, no. 3, 1 May 2018 (2018-05-01), GB, pages 624 - 640, XP055934590, ISSN: 0734-9750, DOI: 10.1016/j.biotechadv.2017.12.009
- GHOSE CHANDRABALI ET AL: "Gram-Negative Bacterial Lysins", vol. 9, no. 2, 11 February 2020 (2020-02-11), pages 74, XP055800842, Retrieved from the Internet <URL:https://www.ncbi.nlm.nih.gov/pmc/articles/PMC7168136/pdf/antibiotics-09-00074.pdf> DOI: 10.3390/antibiotics9020074
- MENG WU, LU HAI-RONG, HUANG QING-SHAN: "Expression and Antibacterial Activity of CHAP Catalytic Domain of Staphylococcus aureus Phage Lysin Ply187", BIOTECHNOLOGY BULLETIN, vol. 32, no. 9, 1 September 2016 (2016-09-01), pages 232 - 238, XP055802963, ISSN: 1002-5464, DOI: 10.13560/j.cnki.biotech.bull.1985.2016.09.031
- WANG XINGLONG; DANG RUIYI; YANG ZENGQI: "The interferon antagonistic activities of the V proteins of NDV correlated with their virulence", VIRUS GENES, vol. 55, no. 2, 31 January 2019 (2019-01-31), KLUWER ACADEMIC PUBLISHERS, BOSTON., US, pages 233 - 237, XP036756072, ISSN: 0920-8569, DOI: 10.1007/s11262-019-01637-3

## Description

### FIELD OF TECHNOLOGY

The present invention relates to a field of hybrid antimicrobial protein.

### BACKGROUND

Antibiotics have been widely used in the fields of medical and health services and agricultural breeding, and have played an important role in treatment of infectious diseases and protection of public health security. However, with extensive use of the antibiotics, bacteria have become increasingly resistant to the antibiotics. It resulted clinical treatment difficulties and increased mortality in the world and therefor seriously threatened human health.

Multi-drug resistant bacteria mainly include *Acinetobacter baumannii, Pseudomonas aeruginosa, Enterobacteriaceae, Enterococcus* and *Staphylococcus aureus,* in which Gram-negative bacteria (G⁻ bacteria) have a largest proportion, and particularly drug resistance of the *Acinetobacter baumannii* has become more and more serious in recent years (Mezzatesta M L, D' Andrea M Mezza Testa, Migliavacca R, et al. Epidemiological characterization and distribution of carbapenem-resistant Acinetobacter baumannii clinical isolates in Italy [J]. Clinical Microbiology and Infection, 2012, 18(2): 160-166. Durante-Mangoni E, Zarrilli R. Global spread of drug-resistant Acinetobacter baumannii: molecular epidemiology and management of antimicrobial resistance [J]. Future microbiology, 2011, 6(4): 407-422.). Among 12 deadliest drug-resistant bacteria ranked by the World Health Organization (WHO), the *Acinetobacter baumannii, Pseudomonas aeruginosa* and *Enterobacteriaceae* which resistant to carbapenem antibiotics are the most serious. It is urgent to develop new antimicrobial drugs, especially antimicrobial drugs with novel action modes different from the traditional antibiotics.

The traditional antibiotics have broad-spectrum antimicrobial properties and take the same effect on normal microbial flora. During extensive use of the antibiotics, the normal microbial flora also envolved drug resistance. As drug-resistant genes are transmitted between the normal microbial flora and pathogenic bacteria, the probability of drug-resistant bacteria is significantly increased. As a main component of a bacterial cell wall, peptidoglycan is a three-dimensional network structure formed by cross-linking a sugar chain, a peptide chain and a peptide bond bridge. The peptidoglycan is used for maintaining the mechanical strength of the cell wall and preventing bacteria from being affected by external factors. As a class of proteins that can specifically lyse the peptidoglycan of the bacterial cell wall, peptidoglycan hydrolases mainly include bacteriophage lysins, bacteriocins and bacterial autolysins (Vollmer W, Joris B, Charlier P, et al. Bacterial peptidoglycan(murein) hydrolases [J]. FEMS microbiology reviews, 2008, 32(2): 259-286.). For example, a *Staphylococcus aureus* bacteriophage lysin LysK, a *Staphylococcus simulans* bacteriocin lysostaphin and a *Streptococcus pneumoniae* autolysin LytA are included. A peptidoglycan hydrolase generally consists of a catalytic structural domain (catalytic domain) used for hydrolyzing the peptidoglycan and a targeting structural domain (binding domain) used for specifically targeting a bacterial cell wall.

When being added into a bacterial solution, the peptidoglycan hydrolase can specifically bind to sensitive bacteria and rapidly lyse cell walls. It has been extensively proved that the peptidoglycan hydrolase has antimicrobial activity in vivo and in vitro (Gerstmans H, Rodriguez-Rubio L, Lavigne R, et al. From endolysins to Artilisin®s: novel enzyme-based approaches to kill drug-resistant bacteria [J]. Biochemical Society Transactions, 2016, 44(1): 123-128. Guo M, Feng C, Ren J, et al. A Novel Antimicrobial Endolysin, LysPA26, against Pseudomonas aeruginosa [J]. Frontiers in Microbiology, 2017, 8:293.). The peptidoglycan hydrolase can quickly kill specific bacteria by directly lysing the peptidoglycan of the bacteria, has a narrow antimicrobial spectrum and a bactericidal mechanism different from the traditional antibiotics, and can be used repeatedly for a long time without easy induction drug resistance. The peptidoglycan hydrolase can directly lyse the bacteria in every growth cycle. By using the narrow-spectrum peptidoglycan hydrolase, the normal microbial flora is hardly affected.

However, there are some problems need to be improved when natural peptidoglycan hydrolases are directly used, for example, low antimicrobial activity, very narrow antimicrobial spectrum, low expression amount, poor solubility. In addition, the combination of the catalytic domain and the binding domain are not in a best condition, or there are other non-essential domains between them. Wild-type LysK includes two catalytic domains (CHAP and Amidase structural domains) while the Amidase structural domain has very low catalytic activity. When the Amidase structural domain is removed from the LysK (LysKΔ221-390), the activity of a protein is not changed (Becker S C, Dong S, Baker J R, et al. LysK CHAP endopeptidase domain is required for lysis of live staphylococcal cells [J]. FEMS microbiology letters, 2009, 294(1): 52-60.). An isolated CHAP catalytic domain of the LysK also has no antimicrobial activity in a TSB culture medium. In a complex environment, the catalytic domain needs to be combined with a binding domain to achieve more effect. However, the binding domain of the LysK has no great effect, and is leading to low affinity efficiency and narrow identification range. Therefore, construction of a highly active hybrid antimicrobial protein has great significance for promoting research and development of novel antimicrobial drugs. It will provide an effective solution for solving the problem of the bacteria of drug resistance.

In 2013, Gladskin, as the first bacteriophage lysin based antimicrobial protein product on the market in the world, is used for treatment of inflammatory skin diseases, such as eczema, rosacea and acne, caused by methicillin-resistant *Staphylococcus aureus* (MRSA) in intact skin. At present, clinical researches have already been conducted on many antimicrobial proteins, such as Lysostaphin (China, used for treatment of a wound infection with the *Staphylococcus aureus*), P128 (India, used for removal of the *Staphylococcus aureus* fixed to the nasal mucosa), CF301 (the United States of America, used for treatment of septicemia caused by the *Staphylococcus aureus*) and SAL200 (South Korea, used for treatment of the septicemia caused by the *Staphylococcus aureus*). In addition, clinical researches have also been conducted on some antimicrobial proteins, such as Cpl-1, ClyS, ClyH and PlyV12, which are used for treatment of infections caused by *Staphylococcus aureus, Streptococcus pneumoniae* and *Enterococcus,* respectively. All these antimicrobial proteins with clinical researches are focused on treatment of infections with G⁺ bacteria, and at present, there are no clinical researches on antimicrobial proteins used for treatment of G⁻ bacteria. Based on the severity of the drug-resistant G⁻bacteria and the imbalance in development of antimicrobial protein drugs, it is urgent to develop new antimicrobial drugs against the G⁻bacteria.

WO 2018/185634 A1 discloses polypeptides comprising an amino acid sequence of a globular Gram-negative endolysin and a cell wall-binding domain. These polypeptides can be used for treating humans or animals through surgery or therapy, as well as for diagnostic purposes. This document further describes corresponding nucleic acids, vectors, bacteriophages, host cells, and compositions, which can also be used as antimicrobial agents in food, cosmetics, or as disinfecting agents.

Oliveira Hugo ET AL ("Structural and Enzymatic Characterization of ABgp46, a Novel Phage Endolysin with Broad Anti-Gram-Negative Bacterial Activity", Frontiers in Microbiology, vol. 7, 26 February 2016 (2016-02-26), XP093083621) describes a phage endolysin (ABgp46) from *Acinetobacter* phage vb_AbaP_CEB1, exhibiting antibacterial activity against Gram-negative pathogens, including multidrug-resistant *Acinetobacter baumannii.* ABgp46 functions as an N-acetylmuramidase, active across a broad pH range and temperatures up to 50°C. It reduces multidrug-resistant *A. baumannii* strains by up to 2 logs within 2 hours. In combination with citric and malic acid, ABgp46 shows enhanced antibacterial effects against A. *baumannii, Pseudomonas aeruginosa,* and *Salmonella typhimurium,* suggesting potential use in human and veterinary medicine.

According to Gerstmans Hans ET AL ("Synthetic biology of modular endolysins", BIOTECHNOLOGY ADVANCES., vol. 36, no. 3, 1 May 2018 (2018-05-01), pages 624-640, XP055934590), endolysins and their derivatives have emerged as a novel class of antibacterials. Their rapid action and proteinaceous nature distinguish them from other antibiotics. A key feature is their modularity, enabling customization of specificity, activity, stability, and solubility. Protein engineering has expanded their activity against drug-resistant Gram-negative bacteria and improved efficacy against Gram-positive pathogens. Additionally, specific cell wall binding domains from endolysins are used for diagnostic development.

### SUMMARY

An objective of the present invention is to provide a hybrid antimicrobial protein having a strong bactericidal effect and an application thereof, so as to overcome the defects of the prior art and meet human needs.

The hybrid antimicrobial protein of the present invention, having a strong bactericidal effect against Gram-negative bacteria, is a hybrid protein with the amino acid sequence of SEQ ID NO: 1 which named AB469, a hybrid protein named AB46M9 with an amino acid sequence shown in SEQ ID NO 3, a hybrid protein named AB469M with an amino acid sequence shown in SEQ ID NO 5, or a hybrid protein named B946 with an amino acid sequence shown in SEQ ID NO 7.

The AB469 includes 285 amino acids and has a molecular weight of 31.8 kD and a theoretical isoelectric point of 9.8. After purification in 2 steps, the purity can reach 95% or above. According to SDS-PAGE electrophoresis, it is shown that the AB469 has a molecular weight of about 32 kD.

A nucleic acid sequence encoding the AB469 is shown in SEQ ID NO: 2 and named ab469.

According to a test, it is proven that the AB469 has high solubility and stability.

According to an in vitro test, it is proven that the AB469 has high activity, a broad spectrum and a strong bactericidal effect on *Acinetobacter baumannii, Pseudomonas aeruginosa, Klebsiella pneumoniae, Salmonella typhi* and *Escherichia coli.* After adding 10 µg/mL of the AB469 into bacterial fluid and acts for 2 h, the *Acinetobacter baumannii* (ATCC 19606), the *Pseudomonas aeruginosa* (ATCC 15442) and the *Salmonella typhi* (CMCC 50094) can be decreased by more than 7 log values, and the *Klebsiella pneumoniae* (ATCC 700603) and the *Escherichia coli* (ATCC 35218) are decreased by more than 2 log values. After adding 50 µg/mL of the AB469 acts for 2 h, the *Klebsiella pneumoniae* (ATCC 700603) and the *Escherichia coli* (ATCC 35218) can be decreased by more than 7 log values, and *Enterobacter cloacae* (ATCC 700323) and *Staphylococcus aureus* (ATCC 6538) are decreased by about 4 log values.

The antimicrobial protein AB469 can effectively kill the clinically isolated *Acinetobacter baumannii,* the *Pseudomonas aeruginosa* and the *Klebsiella pneumoniae.* In addition, the AB469 has the same effect on the *Acinetobacter baumannii,* the *Pseudomonas aeruginosa* and the *Klebsiella pneumoniae* with multi-drug resistance (to ceftazidime, ceftriaxone, meropenem, imipenem, tobramycin and sulbactam).

The AB469 also has high activity under complex conditions such as serum and body fluid.

A nucleic acid sequence encoding the AB46M9 is shown in SEQ ID NO: 4 and named ab46m9.

A nucleic acid sequence encoding the AB469M is shown in SEQ ID NO: 6 and named ab469m.

A nucleic acid sequence encoding the B946 is shown in SEQ ID NO: 8 and named b946.

Compared with wild-type ABgp46, the AB469 has one more binding domain, the antimicrobial activity in a complex environment can be significantly improved, and the complex environment includes certain ionic strength, a culture medium, serum and a body fluid.

According to a test, it is shown that under certain ionic strength (150 mM NaCl), the bactericidal activity of the ABgp46 is significantly reduced (Table 1), and a log decrease value is decreased from 5.58 to 1.54. The results are consistent with those of many proteins with only one catalytic domain (Osipovitch D C, Griswold K E. Fusion with a cell wall binding domain renders autolysin LytM a potent anti-Staphylococcus aureus agent [J]. FEMS microbiology letters, 2015, 362(2):1-7.). From Table 1, it can be seen that the effect of the ionic strength on the ABgp46 is significantly higher than that on the AB469. A bactericidal effect of Oliverira-expressed ABgp46 (2µM, ~40 µg/mL) on the *Pseudomonas aeruginosa* (decreased by 4 log values) is lower than that on the *Acinetobacter baumannii* (decreased by more than 5 log values). The AB469 of the present invention with a lower protein concentration (0.31 µM, 10 µg/mL), has a strong bactericidal effect on the *Acinetobacter baumannii* and the *Pseudomonas aeruginosa* under 150 mM NaCl (decreased by more than 7 log values).

The ABgp46 is a bacteriophage lysin only including one catalytic domain and has been reported in detail in the following literature: Oliveira H, Boas D V, Mesnage S, et al. Structural and enzymatic characterization of ABgp46, a novel phage endolysin with broad anti-gram-negative bacterial activity [J]. Frontiers in microbiology, 2016, 7.

The hybrid antimicrobial protein having a strong bactericidal effect can be used for preparing an antimicrobial preparation. The antimicrobial preparation includes an antimicrobial effective amount of the hybrid antimicrobial protein having a strong bactericidal effect on Gram-negative bacteria and a drug carrier. The drug carrier includes an excipient, such as water and hydroxypropyl methyl cellulose.

Preferably, in the antimicrobial preparation, the hybrid antimicrobial protein having a strong bactericidal effect on Gram-negative bacteria has a weight percentage of 0.0001-0.05%.

The antimicrobial preparation can be applied to a surface of a human body or an object in need by spraying.

The present invention has the following beneficial effects.

In the present invention, on the basis of a large number of researches, a new hybrid antimicrobial protein is successfully constructed. The *Escherichia coli* or yeast is used for expression, and after purification, the hybrid antimicrobial protein with high purity is obtained. The protein can rapidly lyse the Gram-negative bacteria, such as the *Acinetobacter baumannii,* the *Pseudomonas aeruginosa,* the *Klebsiella pneumoniae,* the *Salmonella typhi* and the *Escherichia coli.* The antimicrobial preparation prepared from the antimicrobial protein can be used for prevention and treatment of bacterial infection. The antimicrobial protein is a new and efficient antimicrobial substance that can directly lyse bacteria. The antimicrobial protein can be prepared into an antimicrobial preparation used for prevention and treatment of bacterial infection and sterilization of medical apparatus and instruments and medical places.

### BRIEF DESCRIPTION OF THE DRAWINGS

Fig. 1 is an SDS-PAGE diagram of AB469, AB46M9, AB469M and B946.
Fig. 2 shows detection of biological activity of the AB469 against *Acinetobacter baumannii* (ATCC 19606) by using a turbidimetric method.
Fig. 3 shows detection of biological activity of the AB469 against *Pseudomonas aeruginosa* (ATCC 15442) by using a turbidimetric method.
Fig. 4 shows detection of biological activity of the AB469 against *Klebsiella pneumoniae* (ATCC 700603) by using a turbidimetric method.
Fig. 5 shows detection of biological activity of the AB469, the AB46M9, the AB469M and the B946 against the *Acinetobacter baumannii* (ATCC 19606) by using a turbidimetric method.

### DETAILED DESCRIPTION

The present invention is further described below in conjunction with specific embodiments, but the protection scope of the present invention is not limited thereto;
In the present invention, through analysis of a structural domain of an antimicrobial protein, a hybrid protein having high antimicrobial activity against Gram-negative bacteria is artificially designed, and an amino acid sequence is shown in SEQ ID NO: 1. Secretion expression of the protein in *Escherichia coli* is achieved by using a molecular biology method. After purification, a protein with high purity is obtained for activity detection.

### Embodiment 1 Construction, expression and purification of an AB469 engineering bacterium

### 1. Construction of an AB469 engineering bacterium

According to the preference of a codon of *Escherichia coli,* a nucleic acid sequence ab469 (SEQ ID NO: 2) encoding the AB469 was artificially designed and synthesized. A *Ncol* restriction endonuclease site (italic part) was added into a 5' end, and a nucleic acid sequence ct was introduced with g at the end of the restriction site to form a codon encoding an alanine. A *HindIII* restriction endonuclease site was added into a 3' end (italic part). The sequence was as follows:

In order to facilitate expression in the *Escherichia coli* and to form a soluble target protein, Cys in an SP10 binding domain was mutated into Ser. An artificially synthesized ab469 gene inserted to a vector pET28a(+) through being connected between the *Ncol* and *HindIII* sites to obtain a recombinant plasmid pET28a-ab469. The recombinant plasmid was transformed into competent cells of *Escherichia coli* BL21 (DE3) to obtain an engineering bacterium expressing the AB469.

### 2. Expression and purification of AB469

A monoclonal engineering bacterium was inoculated into an LB culture solution (containing 30 mg/L of kanamycin) and subjected to shaking culture overnight at 30°C, then inoculated into the same LB culture solution with a ratio of 1%, and subjected to shaking culture at 30°C until an optical density (wavelength 600 nm) was about 0.6. An IPTG with a final concentration of 0.05 mM was added to induce protein expression and continuously subjected to shaking culture at 30°C, induction was conducted for about 4 h, and a fermentation supernatant was collected by centrifugation.

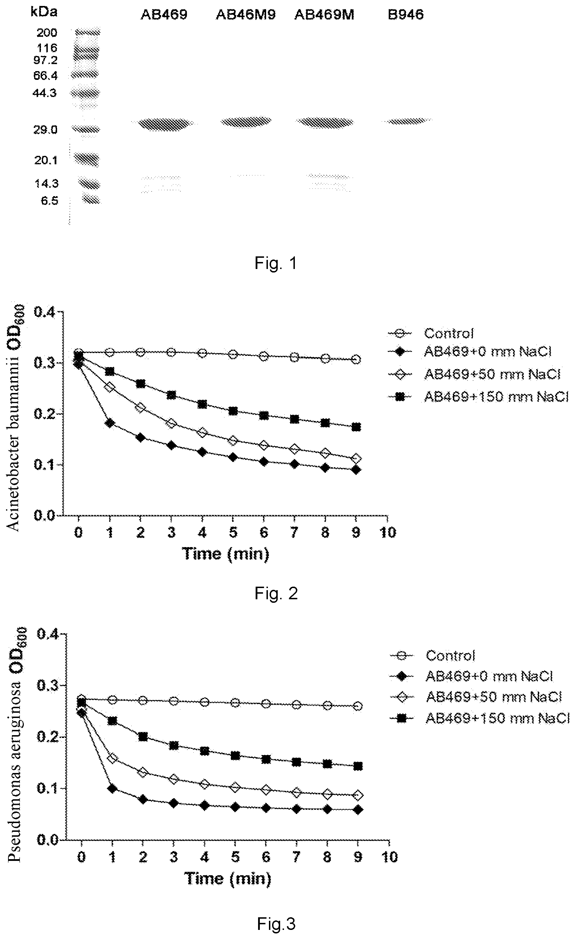

The fermentation supernatant was purified by two steps of cation exchange and gel filtration. A purified sample was cryopreserved at -20°C for later use. The weight and purity of a recombinant protein sample were determined by using 10% SDS-PAGE. According to results, it could be seen that a main electrophoresis band of a recombinant hybrid antimicrobial protein AB469 was about 32 kD (see Fig. 1).

### Embodiment 2 Construction, expression and purification of engineering bacteria of mutants AB46M9, AB469M and B946 of AB469

### 1. Construction of engineering bacteria of mutants

The mutant AB46M9 (SEQ ID NO: 3) consisted of a catalytic domain AB46M (SEQ ID NO: 10) having 82% similarity with an amino acid sequence of AB46 (1-185aa) (SEQ ID NO: 9), a flexible linker, and a binding domain SP10 (154-236aa) (SEQ ID NO: 11), in sequence.

The mutant AB469M (SEQ ID NO: 5) consisted of a catalytic domain AB46 (1-185aa), a flexible linker, and a binding domain B9M (SEQ ID NO: 12) having 82% similarity with an amino acid sequence of SP10 (154-236aa), in sequence.

The B946 (SEQ ID NO: 7) consisted of a binding domain SP10 (154-236aa), a flexible linker in the middle, and a catalytic domain AB46 (1-185aa), in sequence.

According to the preference of a codon of *Escherichia coli,* nucleic acid sequences ab46m9 (SEQ ID NO: 4), ab469m (SEQ ID NO: 6) and b946 (SEQ ID NO: 8) that can encode AB46M9, AB469M and B946 were artificially designed and synthesized.

Construction of expression vectorsand engineering bacteria of the ab46m9, the ab469m and the b946, and specific expression and purification methods of the ab46m9, the ab469m and the b946 were the same as those of the ab469. Purified AB46M9, AB469M and B946 samples were cryopreserved at -20°C for later use. The weight and purity of recombinant protein samples were determined by using 10% SDS-PAGE (see Fig. 1).

### Embodiment 3 Detection of bactericidal activity of AB469 against Acinetobacter baumannii, Pseudomonas aeruginosa and Klebsiella pneumoniae by using a turbidimetric method

The *Acinetobacter baumannii* (ATCC 19606), the *Pseudomonas aeruginosa* (ATCC 15442) and the *Klebsiella pneumoniae* (ATCC 700603) cultured overnight were transferred and allowed to grow to a mid-log phase (OD₆₀₀ was about 0.5). Bacterial bodies were collected by centrifugation (5,000 g, 5 min). The bacteria bodies were washed twice with 20 mM PBS (pH 7.2) and then resuspended in PBS buffers with different concentrations of NaCl (0-600 mM). A sample (to a final concentration of 2 µg/mL) or a PBS control, 10 µl of a 200 mM EDTA (to a final concentration of 0.5 mM) and 50 µl of a bacterial suspension were added into a 96-well plate, and the PBS was supplemented until a total reaction system was 200 µl. A reaction temperature was 37°C, and reading results was conducted at a wavelength of 600 nm (for 10 times at an interval of 1 min).

Turbidimetric detection results were shown in Fig. 2 to Fig. 4. The AB469 could lyse pathogenic bacteria, thereby causing the OD value of a bacterial solution decreased. When the OD value of the bacterial solution of the pathogenic bacteria was rapidly decreased, it was indicated that the AB469 had a rapid bactericidal effect on the *Acinetobacter baumannii* (ATCC 19606), the *Pseudomonas aeruginosa* (ATCC 15442) and the *Klebsiella pneumoniae* (ATCC 700603). With increase of the salt concentration, the AB469 still had high bactericidal activity. The bactericidal activity of the AB469 could be significantly improved by using some non-ionic surfactants (such as BriJ98, Tween 20 and Peregal).

### Embodiment 4 Detection of bactericidal activity of AB46M9, AB469M and B946 against Acinetobacter baumannii by using a turbidimetric method

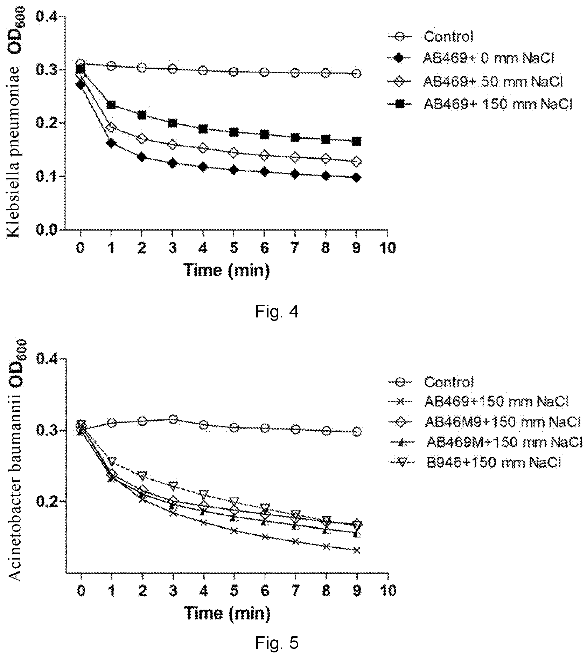

The turbidimetric method was the same as that in Embodiment 3 for detection, and turbidimetric detection results were shown in Fig. 5. Compared with the AB469, the bactericidal activity of the mutants AB46M9, AB469M and B946 against the *Acinetobacter baumannii* was not significantly reduced.

### Embodiment 5 In vitro timed and quantitative killing effects of AB469 on standard strains and clinically isolated strains of pathogenic bacteria

Bacteria cultured overnight were transferred and allowed to grow to a mid-log phase (OD₆₀₀ was about 0.5). Bacterial bodies were collected by centrifugation (5,000 g, 5 min). The bacterial bodies were diluted with PBS (pH 7.2) to about 1*10⁸ cfu/mL. 1 mL of a sample containing the AB469 (to a final concentration of 10-50 µg/mL); NaCl (to a final concentration of 150 mM); and an EDTA (to a final concentration of 0.5 mM) or sodium citrate (to a final concentration of 8 mM) was added into 250 µl of a bacterial solution. After reaction at room temperature for 120 min, 0.5 mL of a sample solution was taken for serial dilution, and then 0.5 mL of a sample solution was taken and subjected to viable count culture. 40 µh/mL of AB46 and an EDTA (to a final concentration of 0.5 mM) were used as a control (after reaction at room temperature for 120 min). A preparation method of the AB46 was the same as that of the AB469.

According to results of an in vitro bactericidal experiment, it was shown that compared with the isolated AB46, the hybrid antimicrobial protein AB469 had stronger activity in killing *Acinetobacter baumannii* (ATCC 19606) (Table 1) and was more resistant to ionic strength.

**Table 1 Comparison of bactericidal activity of ABgp46 and AB469 against Acinetobacter baumannii (ATCC 19606)**

| | 0 mm NaCl | 150 mm NaCl |
|---|---|---|
| PBS/EDTA | 0.24±0.12 | 0.16±0.09 |
| ABgp46 (40 µg/mL)/EDTA | 5.58±0.33 | 1.54±0.23 |
| AB469 (10 µg/mL)/EDTA | >8.21±0.57 | >7.8±0.38 |

According to results of an in vitro bactericidal experiment (Table 2), it was shown that after 10 µg/mL of the AB469 acted for 2 h, the *Acinetobacter baumannii* (ATCC 19606), *Pseudomonas aeruginosa* (ATCC 15442) and *Salmonella typhi* (CMCC 50094) could be effectively killed and decreased by more than 7 log values; *Klebsiella pneumoniae* (ATCC 700603) and *Escherichia coli* (ATCC 35218) decreased by more than 2 log values; *Enterobacter cloacae* (ATCC 700323) and *Staphylococcus aureus* (ATCC 6538) decreased by about 1 log value.

According to results in Table 3, it was shown that after 50 µg/mL of the AB469 acted for 2 h, the *Klebsiella pneumoniae* (ATCC 700603) and the *Escherichia coli* (ATCC 35218) could be effectively killed; the *Enterobacter cloacae* (ATCC 700323) and the *Staphylococcus aureus* (ATCC 6538) decreased by about 4 log values.

**Table 2 Bactericidal activity of AB469 (10 µg/mL)/EDTA against different pathogenic bacteria under 150 mM NaCl**

| | PBS/EDTA | AB469 (10 µg/mL)/EDTA |
|---|---|---|
| *Acinetobacter baumannii* (ATCC 19606) | 0.16±0.09 | >7.84±0.38 |
| *Pseudomonas aeruginosa* (ATCC 15442) | 0.56±0.09 | >7.01±0.27 |
| *Salmonella typhi* (CMCC 50094) | 0.39±0.14 | >7.59±0.34 |
| *Klebsiella pneumoniae (ATCC 700603)* | 0.27±0.15 | 2.15±0.18 |
| *Escherichia coli* (ATCC 35218) | 0.26±0.14 | 2.74±0.13 |
| *Enterobacter cloacae* (ATCC 700323) | 0.17±0.06 | 0.90±0.08 |
| *Staphylococcus aureus* (ATCC 6538) | 0.22±0.11 | 1.34±0.16 |

**Table 3 Bactericidal activity of AB469 (50 µg/mL)/EDTA against different pathogenic bacteria under 150 mM NaCl**

| PBS/EDTA | AB469 (50 µg/mL)/EDTA |
|---|---|
| *Klebsiella pneumoniae* (ATCC 700603) | >7.36±0.28 |
| *Escherichia coli* (ATCC 35218) | >7.62±0.32 |
| *Enterobacter cloacae* (ATCC 700323) | 3.66±0.25 |
| *Staphylococcus aureus* (ATCC 6538) | 4.58±0.27 |

After 10 µg/mL of the antimicrobial protein AB469 acted for 2 h, the clinically isolated *Acinetobacter baumannii* and *Pseudomonas aeruginosa* could be effectively killed (Table 4). The AB469 had the same effect on multi-drug (comprising ceftazidime, ceftriaxone, meropenem, imipenem, tobramycin and sulbactam, etc.) resistant strains. After 50 µg/mL of the antimicrobial protein AB469 acted for 2 h, the clinically isolated *Klebsiella pneumoniae* including multi-drug resistant bacteria could be effectively killed.

**Table 4 Bactericidal activity of AB469/EDTA against different clinically isolated pathogenic bacteria under 150 mM NaCl**

| Pathogenic bacteria | Clinical isolation number | Drug resistance | AB469 (10 µg/mL)/EDTA | AB469 (50 µg/mL)/EDTA |
|---|---|---|---|---|
| *Acinetobacter baumannii* | 1512164 | - | >7.17 | |
| | 1511566 | - | >8.12 | |
| | 1512069 | - | >8.47 | |
| | 1511361 | + | >8.03 | |
| | 1511362 | + | >7.30 | |
| | 1511368 | + | >8.69 | |
| *Pseudomonas aeruginosa* | 1511387 | - | >7.75 | |
| | 1511410 | - | >7.65 | |
| | 1511475 | - | >7.92 | |
| | 1511386 | + | >6.71 | |
| | 1511389 | + | >7.47 | |
| | 1511433 | + | >7.30 | |

| *Klebsiella pneumoniae* | 1511549 | - | >6.78 | |
|---|---|---|---|---|
| | 1511691 | - | >6.90 | |
| | 1511674 | - | >6.60 | |
| | 1511673 | - | >6.89 | |
| | 1511558 | + | >6.95 | |
| | 1511535 | + | >7.08 | |

According to the results above, the hybrid AB469 had a very strong bactericidal effect on Gram-negative bacteria and the same effect on drug-resistant bacteria. The AB469 also had a strong bactericidal effect on the *Staphylococcus aureus* (Gram-positive bacteria).

Fig. 1 was provided.

### Embodiment 6

100 ml of a biological antimicrobial preparation containing AB469 was prepared.

A prescription was as follows:

| | |
|---|---|
| AB469 | 0.02 g |
| Anhydrous potassium dihydrogen phosphate | 0.16 g |
| Anhydrous disodium hydrogen phosphate | 0.12 g |
| Ethylenediaminetetraacetic acid disodium salt | 0.037 g |
| Glycerol | 3 ml |
| NaCl | 0.6 g |
| Water | Supplemented to 100 ml |

A preparation method was as follows:
a) required amounts of auxiliary materials were calculated according to the prescription and the total volume of a preparation solution, accurately weighed and then put into a clean container;
b) the water that was 70% of the total preparation solution was added into a batching vessel to fully dissolve the NaCl, the ethylenediaminetetraacetic acid disodium salt, the disodium hydrogen phosphate and the potassium dihydrogen phosphate first, then the glycerol was added for uniform mixing, the AB469 was added, and finally, the water was added to reach a required volume for uniform mixing;
c) sterilization filtration: a prepared antimicrobial preparation was introduced into a sterilization filter, and a discharged antimicrobial preparation was put into a sterile container; and
d) filling: the sterile antimicrobial preparation was filled in a plastic or glass bottle.

The antimicrobial preparation could be used for sterilization and disinfection of a wound or a wound surface for 1-2 times a day.

### SEQUENCE LISTING

<110> Jiangxi Yuansheng Biological Technology Co., Ltd.
<120> HYBRID ANTIMICROBIAL PROTEIN HAVING STRONG
   BACTERICIDAL EFFECT AND APPLICATION THEREOF
<160> 12
<170> SIPOSequenceListing 1.0
<210> 1
   <211> 285
   <212> PRT
   <213> Artificial Sequence
<400> 1
<210> 2
   <211> 864
   <212> DNA
   <213> Artificial Sequence
<400> 2 CCATTCTGAAAAAACTGTAAGCTT 864
<210> 3
   <211> 285
   <212> PRT
   <213> Artificial Sequence
<400> 3
<210> 4
   <211> 864
   <212> DNA
   <213> Artificial Sequence
<400> 4
<210> 5
   <211> 285
   <212> PRT
   <213> Artificial Sequence
<400> 5
<210> 6
   <211> 864
   <212> DNA
   <213> Artificial Sequence
<400> 6
   CCATGGCTATCCTGACCAAAGATGGCTTTGGCATCATTCGCAACGAA CTGTTTGGCGGCA 60
   AACTGGATCAGACACAGGTGGATGCCATTAATTTTATTGTTGAAAAA GCTACCGAATCTG 120
   GGTTAAGTTATCCGGAAGCGGCCTATCTGTTAGCGACGATCTATCAT GAAACGGGTCTGC 180
   CGAGCGGTTATCGTACCATGCAGCCAATCAAAGAAGCCGGTAGTGA TAATTACCTCCGCT 240
   CTAAAAAATATTATCCGTATATCGGCTATGGCTATGTTCAGCTGACGT GGAAAGAAAATT 300
   ATGGTCGTATTGGTAAACTGATCGGCATCGACTTGATCAAAAATCCG GAAAAAGCCTTAG 360
   AACCGCTGATTGCGATTCAGATTGCCATCAAAGGTATGCTGAATGGT TGGTTTACAGGTG 420
   TGGGCTTTCGTCGCAAACGTCCAGTGAGTAAATATAACAAGCAGCA GTATATTGCTGCAC 480
   GCAATATCATTAATGGTAAAGATAAAGCAGAACTGATCGCGAAATATG CCATCATCTTTG 540
   AACGCGCCCTGCGTTCTCTGGGCTCCAATAGTACCTCTAATAGTAGC ACCAATTCAGGCT 600
   CTACCGGTAAAGTGACACTGCCGAGTGGTGTGATTCGCGTGACCAA ACCGATGGTTCATG 660
   GTGACGATGTGCTGGCAATTCAGAAAGCACTGGCAGCCCTGTATTT TTATCCGGATAAAG 720
   GTGCCAAAGATAATGGTATTGATGGTGTTTATGGTCCGAAAACCGCC AATGCCGTTAAAC 780
   GCTTTCAGTCTGTTAATGGCTTAACAGCAGATGGCATTTATGGCCCG AAAACCCGCGCAG 840
   CCATTGAAGAAAAACTGTAAGCTT 864
<210> 7
   <211> 285
   <212> PRT
   <213> Artificial Sequence
<400> 7
<210> 8
   <211> 864
   <212> DNA
   <213> Artificial Sequence
<400> 8
   CCATGGCTAAAGTGAGTCTGCCGAATCGTGTGATTCGCGTGACCAA ACCGATTGTTCATG 60
   GTAGCGATGTGCTGGCAATTCAGAAAGCACTGTCAAGCCTGTATTTT TATCCGGAAAAAG 120
   GTGCCAAAGATAATGGTTCCGATAGCTATTATGGTCCGAAAACCGCC AATGCCGTTAAAC 180
   GCTTTCAGTCTGTTAATGGCTTAGTTGCAGATGGCGTGTATGGCCC GAAAACCCGCGCAG 240
   CCATTCTGAAAAAACTGGGCTCCAATAGTACCTCTAATAGTAGCACC AATTCAGGCTCTA 300
   CCGGTATCCTGACCAAAGATGGCTTTGGCATCATTCGCAACGAACT GTTTGGCGGCAAAC 360
   TGGATCAGACACAGGTGGATGCCATTAATTTTATTGTTGAAAAAGCT ACCGAATCTGGGT 420
   TAAGTTATCCGGAAGCGGCCTATCTGTTAGCGACGATCTATCATGAA ACGGGTCTGCCGA 480
   GCGGTTATCG TACCATGCAG CCAATCAAAG AAGCCGGTAG TGATAATTAC CTCCGCTCTA 540
   AAAAATATTATCCGTATATCGGCTATGGCTATGTTCAGCTGACGTGGA AAGAAAATTATG 600
   GTCGTATTGGTAAACTGATCGGCATCGACTTGATCAAAAATCCGGAA AAAGCCTTAGAAC 660
   CGCTGATTGCGATTCAGATTGCCATCAAAGGTATGCTGAATGGTTGG TTTACAGGTGTGG 720
   GCTTTCGTCGCAAACGTCCAGTGAGTAAATATAACAAGCAGCAGTAT ATTGCTGCACGCA 780
   ATATCATTAATGGTAAAGATAAAGCAGAACTGATCGCGAAATATGCCA TCATCTTTGAAC 840
   GCGCCCTGCGTTCTCTGTAAGCTT 864
<210> 9
   <211> 186
   <212> PRT
   <213> AB46 1-185 aa
<400> 9
<210> 10
   <211> 185
   <212> PRT
   <213> Artificial Sequence
<400> 10
<210> 11
   <211> 83
   <212> PRT
   <213> SP10 154-236 aa
<400> 11
<210>12
   <211> 83
   <212> PRT
   <213> Artificial Sequence
<400> 12

## Claims

1. A hybrid antimicrobial protein having a strong bactericidal effect, wherein the hybrid antimicrobial protein is:
1) a hybrid protein named AB469 with the amino acid sequence shown in SEQ ID NO 1;
2) a hybrid protein named AB46M9 with the amino acid sequence shown in SEQ ID NO 3;
3) a hybrid protein named AB469M with the amino acid sequence shown in SEQ ID NO 5; or
4) a hybrid protein named B946 with the amino acid sequence shown in SEQ ID NO 7.

2. A nucleic acid sequence, wherein the nucleic acid sequence encodes the AB469 of claim 1, is shown in SEQ ID NO: 2 and named ab469.

3. A nucleic acid sequence, wherein the nucleic acid sequence encodes the AB46M9 of claim 1, is shown in SEQ ID NO: 4 and named ab46m9.

4. A nucleic acid sequence, wherein the nucleic acid sequence encodes the AB469M of claim 1, is shown in SEQ ID NO: 6 and named ab469m.

5. A nucleic acid sequence, wherein the nucleic acid sequence encodes the B946 of claim 1, is shown in SEQ ID NO: 8 and named b946.

6. A use of the hybrid antimicrobial protein of claim 1 in preparation of an antimicrobial preparation.

7. An antimicrobial preparation, comprising an antimicrobial effective amount of the hybrid antimicrobial protein of claim 1 and a drug carrier.

## Patentansprüche

1. Hybrides antimikrobielles Protein mit einer starken bakteriziden Wirkung, wobei das hybride antimikrobielle Protein:
1) ein Hybridprotein mit der Bezeichnung AB469 mit der in SEQ ID NO 1 gezeigten Aminosäuresequenz;
2) ein Hybridprotein mit der Bezeichnung AB46M9 mit der in SEQ ID NO 3 gezeigten Aminosäuresequenz;
3) ein Hybridprotein mit der Bezeichnung AB469M mit der in SEQ ID NO 5 gezeigten Aminosäuresequenz; oder
4) ein Hybridprotein mit der Bezeichnung B946 mit der in SEQ ID NO 7 gezeigten Aminosäuresequenz ist.

2. Nukleinsäuresequenz, wobei die Nukleinsäuresequenz das AB469 nach Anspruch 1 kodiert, in SEQ ID NO: 2 gezeigt und als ab469 bezeichnet ist.

3. Nukleinsäuresequenz, wobei die Nukleinsäuresequenz das AB46M9 nach Anspruch 1 kodiert, in SEQ ID NO: 4 gezeigt und als ab46m9 bezeichnet ist.

4. Nukleinsäuresequenz, wobei die Nukleinsäuresequenz das AB469M nach Anspruch 1 kodiert, in SEQ ID NO: 6 gezeigt und als ab469m bezeichnet ist.

5. Nukleinsäuresequenz, wobei die Nukleinsäuresequenz das B946 nach Anspruch 1 kodiert, in SEQ ID NO: 8 gezeigt und als b946 bezeichnet ist.

6. Verwendung des hybriden antimikrobiellen Proteins nach Anspruch 1 bei der Herstellung eines antimikrobiellen Präparats.

7. Antimikrobielles Präparat, umfassend eine antimikrobiell wirksame Menge des hybriden antimikrobiellen Proteins nach Anspruch 1 und einen Arzneimittelträger.

## Revendications

1. Protéine antimicrobienne hybride ayant un fort effet bactéricide, dans laquelle la protéine antimicrobienne hybride est :
1) une protéine hybride nommée AB469 avec la séquence d'acides aminés montrée dans SEQ ID NO : 1 ;
2) une protéine hybride nommée AB46M9 avec la séquence d'acides aminés montrée dans SEQ ID NO : 3 ;
3) une protéine hybride nommée AB469M avec la séquence d'acides aminés montrée dans SEQ ID NO : 5 ; ou
4) une protéine hybride nommée B946 avec la séquence d'acides aminés montrée dans SEQ ID NO : 7.

2. Séquence d'acide nucléique, dans laquelle la séquence d'acide nucléique code pour AB469 selon la revendication 1, est montrée dans SEQ ID NO : 2 et nommée ab469.

3. Séquence d'acide nucléique, dans laquelle la séquence d'acide nucléique code pour AB46M9 selon la revendication 1, est montrée dans SEQ ID NO : 4 et nommée ab46m9.

4. Séquence d'acide nucléique, dans laquelle la séquence d'acide nucléique code pour AB469M selon la revendication 1, est montrée dans SEQ ID NO : 6 et nommée ab469m.

5. Séquence d'acide nucléique, dans laquelle la séquence d'acide nucléique code pour B946 selon la revendication 1, est montrée dans SEQ ID NO : 8 et nommée b946.

6. Utilisation de la protéine antimicrobienne hybride selon la revendication 1 dans la préparation d'une préparation antimicrobienne.

7. Préparation antimicrobienne, comprenant une quantité efficace antimicrobienne de la protéine antimicrobienne hybride selon la revendication 1 et un support médicamenteux.
